# EUROPEAN PATENT SPECIFICATION

(11) **EP 4 504 302 B1**
(45) Date of publication and mention of the grant of the patent: **10.12.2025**
(21) Application number: 23712540.6
(22) Date of filing: 21.03.2023
(51) Int. Cl.: A61M 5/28, A61M 5/32, A61M 5/24

(54) **MULTIPLE INJECTION SYRINGE WITH RETRACTABLE NEEDLE AND ASSISTED NEEDLE ACTUATION FOR INJECTION AND RETURN**
MEHRFACHINJEKTIONSSPRITZE MIT EINZIEHBARER NADEL UND UNTERSTÜTZTER NADELBETÄTIGUNG FÜR INJEKTION UND RÜCKZUG
SERINGUE À INJECTION MULTIPLE AVEC AIGUILLE RÉTRACTABLE ET ACTIONNEMENT ASSISTÉ DE L'AIGUILLE POUR L'INJECTION ET LA RETRACTATION

(30) Priority: 04.04.2022 IT 202200006635
(43) Date of publication of application: 12.02.2025
(73) Proprietor: Università degli studi di Bergamo, 24129 Bergamo (IT)
(72) Inventor: BARAGETTI, Sergio, 24030 Pontida (BG) (IT); PALEARI, Stefano, 20863 Concorezzo (MB) (IT); ARCIERI, Emanuele Vincenzo, 24121 Bergamo (BG) (IT)
(74) Representative: Mittler, Andrea
(86) International application number: PCT/EP2023/057222
(87) International publication number: WO 2023/194097

(56) References cited:
- GB-A- 457 152
- US-A- 5 338 304
- US-A- 5 407 431

## Description

The present invention relates to a multiple injection syringe with retractable needle.

More particularly, the invention relates to a multiple injection syringe with hypodermic safety needle allowing the injection by assisted actuation, and retracts into a container when not in use, thus preventing inadvertent contact with the needle.

Injection syringes of the prior art have hollow needles extending through a plastic hub. To prevent users from accidentally puncturing themselves with the needle tip, the needle is covered by a removable cover. Such covers frictionally engage the plastic hub and can be easily removed once the needle is attached to a syringe cylinder. After use, the cover can be reattached to the needle assembly, which is then separated from the syringe cylinder and discarded. However, there is an unacceptable risk of accidental injury resulting from contact with the needle tip. This is particularly dangerous, as biological fluids contaminating the needle could enter the user's bloodstream. An improved means for covering a used injection needle is needed.

Various syringes with needles having means for protecting a syringe needle from accidental contact with a user's fingers are known from the prior art.

For example, patent US 10661026B2 describes a safety needle device having a housing configured to be coupled to a syringe, the housing having a proximal end, a distal end and a housing body. A needle hub is arranged on the proximal end of the housing and a needle cannula is fixed to the needle hub. The device has a retractable sheath configured to move between an initial position, a retracted position, and an extended position with respect to the housing, in which the initial position partially exposes a distal tip of the needle cannula, the retracted position completely exposes the needle cannula, and the extended position completely covers the distal tip of the needle cannula. The safety needle device can include an activation latch, a locking latch, a cable, and a spring element for pushing the retractable sheath to an extended state to cover the distal end of the needle cannula upon the completion of an injection. The safety needle device can include a slider element arranged above an activation latch.

US-2021/0299361 describes a syringe with a protective housing for the needle.

US-5407431 discloses a syringe with needle of retractable type with assisted actuation suitable for multiple intravenous or intramuscular administration of fluids and for the reentry of the needle in order to avoid accidental punctures

Disadvantageously, the known protection solutions are complex for experienced users to implement and use, and are expensive.

In view of the prior art, it is the object of the present invention to provide a syringe with a retractable needle which ensures the user a safe use without accidental interactions with the needle tip, and the assisted actuation of the needle for multiple injections and the return thereof.

It is a further object of the present invention that the syringe has a simple and inexpensive needle movement mechanism.

It is still a further object of the present invention that the coupling between a tube vial and the needle is facilitated without risks of compromising the integrity of the fluid contained in the tube vial.

According to the present invention, said and further objects are achieved by a syringe as defined in claim 1.

Advantageously, at the end of the injection operations the needle is completely inside the tube vial so as to avoid accidental punctures of the user.

The syringe allows performing multiple injections with a single tube vial, always avoiding the accidental puncture of the user with the needle.

The presence of the springs allows the multiple injection and assisted actuation of the needle for the injection and return into the supporting frame.

It is essential that the tube vial is balanced in the body of the syringe, between head and bottom, and that there is at least one flexural spring for locking/unlocking the axial movement of the tube vial in the frame.

The features and advantages of the present invention will become apparent from the following detailed description of a practical embodiment thereof, shown by way of non-limiting example in the accompanying drawings, in which:
figure 1 shows a perspective view of a syringe according to the present invention;
figure 2 shows an axial sectional view of the syringe;
figure 3 shows an axial sectional view of a needle;
figure 4 shows an enlarged perspective view of the tip of the needle in figure 3;
figure 5 shows an axial sectional view of the syringe in a first configuration, in which a flexural spring is in a first position;
figure 6 shows an axial sectional view of the syringe in a second configuration, in which the flexural spring is in a second position;
figure 7 shows an axial sectional view of the syringe in a third configuration, in which the flexural spring is in the second position;
figure 8 shows an axial sectional view of the syringe in a fourth configuration, in which the flexural spring is in a third position;
figure 9 shows an axial sectional view of the syringe in a fifth configuration, in which the flexural spring is in the second position;
figure 10 shows an axial sectional view of the syringe in a sixth configuration, in which the flexural spring is in the second position;
figure 11 shows an axial sectional view of the syringe in a seventh configuration, in which the flexural spring is in the first position;
figure 12 shows an axial sectional view of the syringe according to a second embodiment.

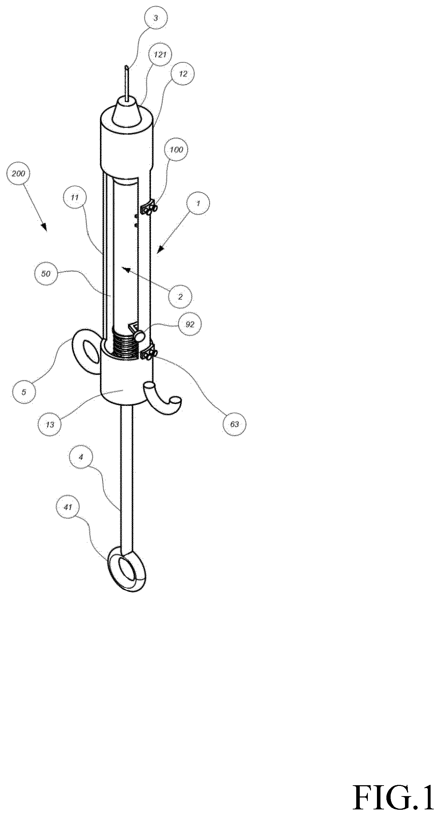

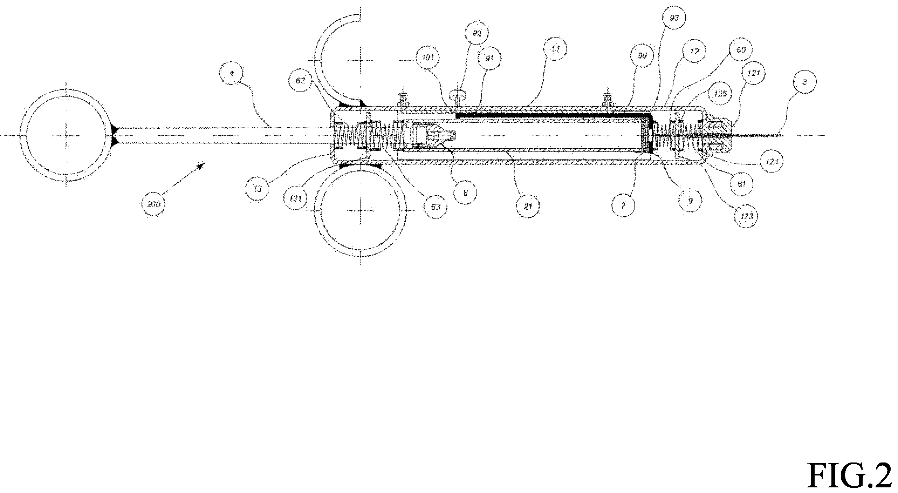

A syringe 200 with a needle 3 of the retractable type with assisted actuation for the injection and return of the needle 3 according to the present invention is suitable for the multiple intravenous or intramuscular administration of fluids. The syringe 200 comprises a hollow-shaped frame 1 adapted to support a tube vial 2 containing a fluid to be administered intravenously or intramuscularly (figures 1-2).

The frame 1 has a cylindrical body 11 for supporting the tube vial 2 and has a head 12 and a bottom 13 at the ends.

The head 12 comprises an end 121, adapted to support the hypodermic-type needle 3 adapted to exit from a hole of the end 121, and a cylindrical cavity in which two springs 60, 61, a movable perforated plate 123 and an end sleeve 124 are at least partially housed.

The movable perforated plate 123 is interposed between the two springs 60, 61, in which the front end of the spring 61 is housed in the end sleeve 124 which is fixed to an internal front part of the head 12 below the end 121. The movable perforated plate 123 is adapted to go to a position whereby the end sleeve 124 abuts on a sleeve 125 placed on the perforated plate 123 on the side of the sleeve 124, as will be clearer below.

The bottom 13 has a through hole for a stem 4 to slide, which ends with a handle 41, and a cylindrical cavity adapted to at least partially house two springs 62, 63 and a movable perforated plate 131 interposed between the two springs 62, 63.

The rear end of the spring 63 is fixed to the movable perforated plate 13, while the front end of the spring 63 is adapted to abuttingly receive the rear end of the tube vial 2, as will be clearer below. The spring 62 is interposed between the movable perforated plate 13 and an internal rear support part of the bottom 13 around the through hole of the bottom 13.

The body 11 is opened laterally to facilitate the assembly/disassembly of the tube vial 2 in a cavity or barrel 50 of the body 11, and comprises two finger grips 5 opposite to each other.

The tube vial 2 comprises a preferably cylindrical, transparent body 21 having the front end closed by a diaphragm 7 made of rubbery material, preferably of medical silicone material; said diaphragm 7 brings the needle 3 in fluid connection with the tube vial 2.

The rear end of the tube vial 2 is closed by a plunger 8 on the rear of which the head of the stem 4 abuts, in which the plunger 8 slides inside the transparent body 21. The transparent body 21 is adapted to rest on the spring 63 so as to be housed in balance in the barrel 50.

The tube vial 2 is of known type and substantially consists of the transparent body 21, the diaphragm 7 and the plunger 8.

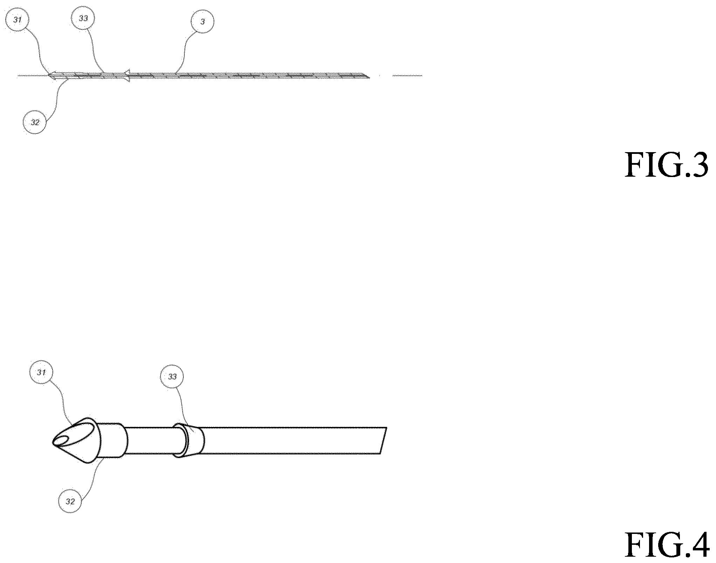

As seen in figures 3 and 4, the needle 3 comprises a conical end 31 cut with a plane transverse to the longitudinal axis of the needle 3 to allow piercing the diaphragm 7 with low pressure. The needle 3 first has a shape match with the diaphragm 7 through a cylindrical section 32 of greater diameter as compared to the main diameter of the needle 3, and a truncated cone portion 33. When the entire tube vial 2 is emptied, the plunger 8 is coupled to the rear end of the needle 3 as set out in the granted Italian patent no. 102016000010073. The syringe 200 can have different shapes of the needle 3, said transverse plane facilitating the piercing of the diaphragm 7.

The syringe 200 comprises a plate 9 which lies orthogonal to the axial direction of motion of the stem 4, is preferably circular in shape, and is perforated in the center for the passage of the needle 3.

Axial direction generally means that of motion of the stem 4 coinciding with the longitudinal axis of the needle 3, the body 11, the tube vial 2 and more generally the syringe 200.

The plate 9 is integral with a flexural spring 90 having the shape of a rod extending in the axial direction.

The spring 90 is fixed to the plate 9 at a front end 93, while at a rear end of the spring 90 it preferably has a knob 92 which allows the user to simply flex the spring 90 in a radial direction, i.e., transverse to the axial direction, and in a circumferential direction with respect to the frame 1 for a locking/unlocking function which will be clearer below. In essence, the knob 92 rotates about two axes orthogonal to the axial direction and orthogonal to each other, around a pin defined substantially close to the fixing point of the spring 90 to the plate 9, i.e., at the upper end 93 of the spring 90.

The syringe 200 further comprises a support 100 for locking the spring 90.

The support 100 is integral with the body 11, has a rod shape similar to that of the spring 90, and has a plurality of cavities 101 each shaped to engage at least one tooth 91 of the spring 90 so as to lock the spring 90 in a desired position inside the frame 1.

The tooth 91 is preferably positioned close to the knob 92 so as to facilitate the coupling/uncoupling.

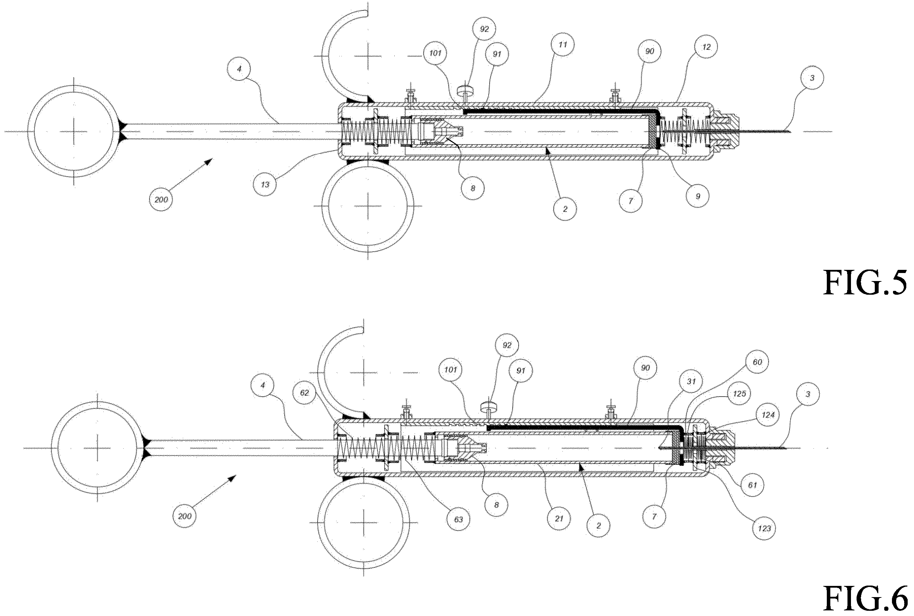

The operation of the syringe 200 is as follows (figures 5-11).

First, the tube vial 2 is inserted into the barrel 50 in which the needle 3 is then inserted.

The tube vial 2 is balanced between the pair of springs 60, 61 and the pair of springs 62, 63, in which the cylindrical cavities of the head 12 and the bottom 13 help the positioning of the tube vial 2 and the maintenance of said balance.

In the initial position (figure 5), the needle 3 partially exits from the end 121 and the knob 92 is in a first position and is maintained in that position by the engagement of the tooth 91 of the spring 90 with a cavity 101 of the plurality of cavities 101.

To be able to do the injection, the user unlocks the plate 9 by acting on the knob 92 radially inwards and in a circumferential direction so as to disengage the tooth 91 from the cavity 101; the user then moves the spring 90 in the axial direction and then the plate 9 towards the front part of the frame 1. A compression of the springs 60, 61 by the plate 9 and an axial displacement towards the front of the tube vial 2 are thus determined, with consequent piercing of the diaphragm 7 by the needle 3. The movable perforated plate 123 reaches a position whereby the end sleeve 124 abuts the sleeve 125 located on the perforated plate 123 on the side of the sleeve 124.

The user acts on the knob 92 again in the radial direction and in the circumferential direction, but in the opposite direction with respect to before, to lock the spring 90 with the engagement of the tooth 91 with another cavity 101 of the support 100 in a second position (figure 6).

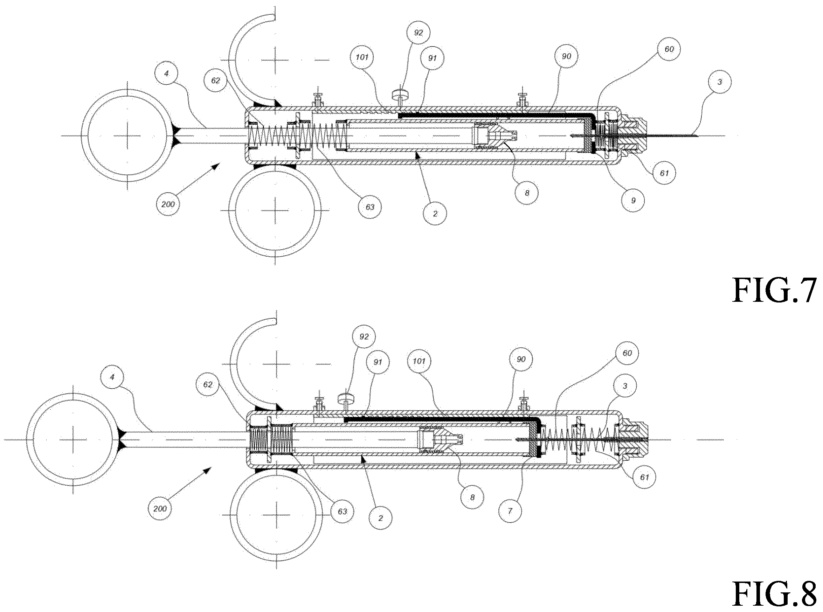

With the spring 90 in the second position, and therefore the plate 9 locked, the user acts on the stem 4, which pushes the plunger 8, to do the injection and inoculate a part of the fluid contained in the tube vial 2 into a tissue (figure 7); this implies a partial emptying of the tube vial 2.

The user then acts again on the knob 92 to release the spring 90 from the engagement of the tooth 91 in the cavity 101 of the support 100, imposing the retraction of the tube vial 2, the spring 90, the plate 9 and the knob 92 to a third position (figure 8), the motion being facilitated by the action of the springs 60, 61, 62 and 63.

In the third position, the needle 3 is completely inside the frame 1, i.e., it does not protrude from the end 121, so as to avoid accidental punctures to the user.

The spring can now be locked in the third position by engaging the tooth 91 with a cavity 101 of the support 100. In said third position, the syringe 200 is ready for new injections so as to completely empty the tube vial 2.

To do a new injection, the user acts on the knob 92 so as to disengage the tooth 91 from the cavity 101 and move the knob 92, the plate 9 and the tube vial 2 towards the front part of the frame 1 as already done for the partial injection described above; similarly, a compression of the springs 60, 61 by the plate 9 is determined, as well as the consequent new exit of the needle 3 from the end 121 of the head 12.

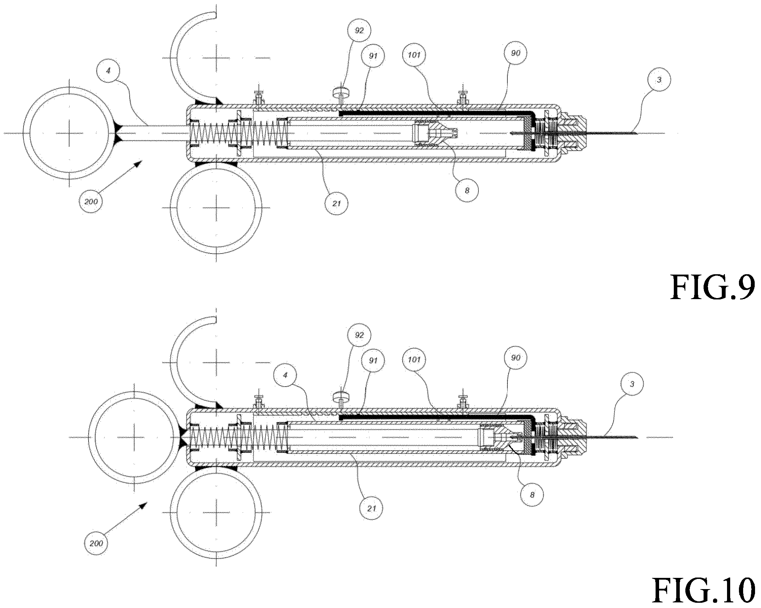

The user again locks the spring 90 and then the plate 9 in the second position (figure 9): the user can again move the stem 4 towards the front part of the syringe 200 to do more injections up to the last injection and inoculate the remaining part of fluid contained in the tube vial 2 into a tissue (figure 10); this implies emptying the tube vial and engaging the plunger 8 with the needle 3 in a known manner as described above with reference to granted Italian patent no. 102016000010073.

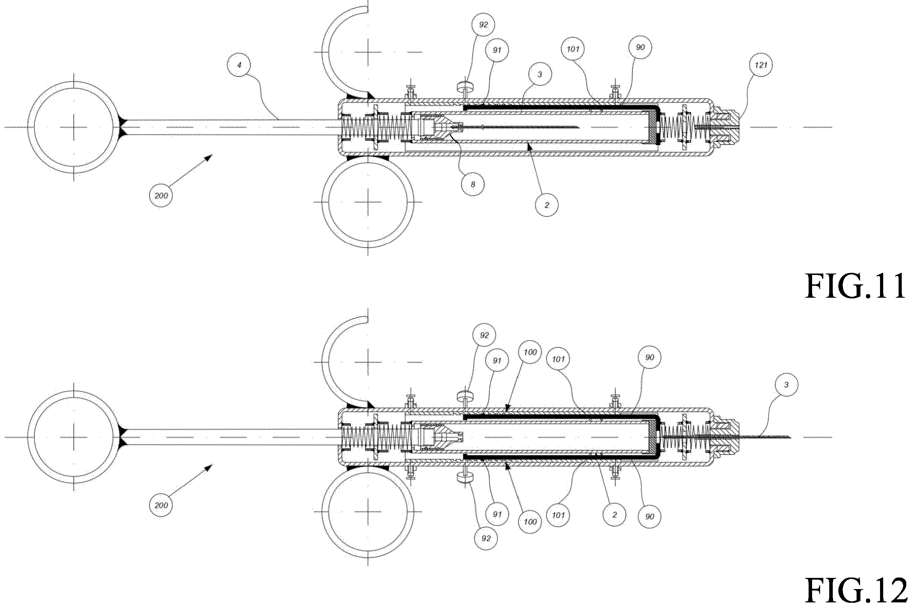

Once the tube vial 2 has been emptied, the spring 90 is returned to the first position to allow the safe replacement of the tube vial 2 and the needle 3 (figure 11).

Advantageously, at the end of the injection, the needle 3 is completely inside the tube vial 2 so as to avoid accidental punctures of the user.

The syringe 200 according to the present invention allows performing multiple injections with a single tube vial 2 always avoiding the accidental puncture of the user with the needle 3.

The presence of the springs 60-63, 90 allows the assisted actuation for the multiple injection and the return of the needle 3 into the frame 1.

Alternative solutions involve the use of two springs 90 (figure 12), only one of the springs 60, 61 and/or only one of the springs 62, 63.

It is possible to provide one or more springs 60-63 only in the head 12 or only in the bottom 13: in the head 12 or in the bottom without springs 60-63, a guide is sufficient.

In essence, it is essential that the tube vial 2 is balanced in the barrel 50, between head 12 and bottom 13, and that there is at least one flexural spring for locking/unlocking the axial movement of the tube vial 2 in the barrel 50 of the frame 1.

## Claims

1. Syringe (200) with needle (3) of retractable type with assisted actuation suitable for multiple intravenous or intramuscular administration of fluids and for the reentry of the needle (3) in order to avoid accidental punctures, comprising a supporting frame (1) of hollow shape adapted to support a tube vial (2) containing the fluid to be administered, and a stem (4) ending with a handle (41), slidingly supported by the supporting frame (1) and adapted to axially move a plunger (8) of the tube vial (2) housed in a transparent body (21) of the tube vial (2) which also has a diaphragm (7) made of rubbery material capable of being pierced by the needle (3),
wherein the supporting frame (1) has a body (11) adapted to support the tube vial (2) in a cavity (50) of the body (11), and which has a head (12) and a bottom (13) at the ends,
wherein the head (12) comprises an end (121) adapted to support the needle (3) which is adapted to exit from a hole of the end (121) of the head (12), and the bottom (13) comprises a through hole for the stem (4) to slide,
wherein at least the head (12) or the bottom (13) have a cylindrical cavity in which at least partially at least one spring (60-63) is housed,
wherein the syringe (200) further comprises
a plate (9) slidably housed in the axial direction in the body (11), adjacent to the diaphragm (7) of the tube vial (2), lying orthogonally to the axial direction of motion of the stem (4), and drilled in the center for the passage of the needle (3),
**characterized in that** the syringe (200) further comprises
at least one flexural spring (90) which extends axially and which is fixed to the plate (9) at a front end (93), wherein the flexural spring (90) is flexible in the radial direction and in the circumferential direction for a function for locking/unlocking of the axial movement of the flexural spring (90),
a support (100) for axial locking of the flexural spring (90), wherein the support (100) is integral with the body (11), extends axially in a similar way to the flexural spring (90) and has a plurality of cavities (101) each shaped to engage with at least one tooth (91) of the flexural spring (90) so as to lock the flexural spring (90) and therefore the plate (9) in a desired position inside the supporting frame (1).

2. Syringe (200) according to claim 1, **characterized in that** the flexural spring (90) is adapted to assume at least
a first locking position in which the needle (3) partially protrudes from the end (121) of the head (12), without fluid connection in the initial positioning phase of the tube vial (2)
a second locking position suitable for allowing the injection of a part of the fluid contained in the tube vial (2) with partial emptying of the tube vial (2), and
a third locking position suitable for the complete reentry of the needle (3) inside the frame (1), and subsequently new outputs of the needle (3) from the end (121) of the head (12) for the execution of further injections to completely empty the tube vial (2).

3. Syringe (200) according to claim 2, **characterized in that** the head (12) comprises a cylindrical cavity in which at least partially at least one front spring (60, 61) is housed, and that the bottom (13) comprises a cylindrical cavity adapted to at least partially house at least one rear spring (62, 63).

4. Syringe (200) according to claim 3, **characterized in that** the cylindrical cavity of the head (12) is adapted to at least partially house two front springs (60, 61), a movable perforated plate (123) and an end sleeve (124), wherein the movable perforated plate (123) is interposed between the two front springs (60, 61), wherein the front end of a front spring (61) is housed in the end sleeve (124) which is fixed to an internal front part of the head (12) under the end (121), and wherein the movable perforated plate (123) is adapted to go into a position whereby the end sleeve (124) abuts on a sleeve (125) placed on the movable perforated plate (123) on the side of the end sleeve (124).

5. Syringe (200) according to claim 3 or 4, **characterized in that** the cylindrical cavity of the bottom (13) is adapted to house at least partially two rear springs (62, 63) and a movable perforated plate (131) placed between the two rear springs (62, 63), wherein the rear end of the first rear spring (63) is fixed to the movable perforated plate (131), while the front end of the first rear spring (63) is adapted to receive in support the rear end of the tube vial (2), wherein the second rear spring (62) is interposed between the movable perforated plate (131) and an internal rear support part of the bottom (13) around the through hole of the bottom (13).

6. Syringe (200) according to any one of the preceding claims, **characterized in that** the needle (3) comprises a conical end (31), a cylindrical portion (32) with a diameter greater than the prevailing diameter of the needle (3), and a truncated cone portion (33), wherein the conical end (31) is cut with a plane transversal to the longitudinal axis of the needle (3) to allow piercing of the diaphragm (7) with a low pressure.

7. Syringe (200) according to any one of the preceding claims, **characterized in that** at a rear end the flexural spring (90) has a knob (92) which allows the user to flex the flexural spring (90).

8. Syringe (200) according to claim 7, **characterized in that** at least one tooth (91) of the flexural spring (90) is positioned in correspondence of the knob (92).

## Patentansprüche

1. Spritze (200) mit Nadel (3) vom einziehbaren Typ, mit unterstützter Betätigung, geeignet für die mehrfache intravenöse oder intramuskuläre Verabreichung von Fluiden und für das Wiedereinführen der Nadel (3), um versehentliche Einstiche zu vermeiden, aufweisend einen hohlen Trägerrahmen (1), der dazu ausgebildet ist, eine Röhrchenampulle (2) abzustützen, die das zu verabreichende Fluid enthält, sowie einen Schaft (4), der in einem Griff (41) endet, durch den Trägerrahmen (1) verschiebbar abgestützt ist und dazu ausgebildet ist, einen Kolben (8) der Röhrchenampulle (2) axial zu bewegen, der in einem transparenten Körper (21) der Röhrchenampulle (2) aufgenommen ist, der außerdem eine Membran (7) aus gummiartigem Material aufweist, die von der Nadel (3) durchstochen werden kann,
wobei der Trägerrahmen (1) einen Körper (11) aufweist, der dazu ausgebildet ist, die Röhrchenampulle (2) in einem Hohlraum (50) des Körpers (11) abzustützen, und der an den Enden einen Kopf (12) und einen Boden (13) aufweist,
wobei der Kopf (12) ein Ende (121) aufweist, das zum Abstützen der Nadel (3) ausgebildet ist, die aus einer Öffnung am Ende (121) des Kopfes (12) austreten kann, und wobei der Boden (13) eine Durchgangsöffnung für die Verschiebung des Schafts (4) aufweist,
wobei dass zumindest der Kopf (12) oder der Boden (13) einen zylindrischen Hohlraum aufweist, in dem mindestens eine Feder (60-63) zumindest teilweise untergebracht ist,
wobei die Spritze (200) ferner eine Platte (9) aufweist, die in dem Körper (11) in axialer Richtung verschiebbar untergebracht ist, benachbart der Membran (7) der Röhrchenampulle (2) und orthogonal zu der axialen Bewegungsrichtung des Schafts (4) angeordnet ist und in der Mitte für die Passage der Nadel (3) gebohrt ist,
**dadurch gekennzeichnet, dass** die Spritze (200) ferner aufweist:
mindestens eine Biegefeder (90), die sich axial erstreckt und an einem vorderen Ende (93) an der Platte (9) befestigt ist, wobei die Biegefeder (90) in radialer Richtung und in Umfangsrichtung flexibel ist, um eine Funktion zum Verriegeln/Entriegeln der axialen Bewegung der Biegefeder (90) auszuführen,
eine Halterung (100) zur axialen Verriegelung der Biegefeder (90), wobei die Halterung (100) einstückig mit dem Körper (11) ausgebildet ist, sich axial in ähnlicher Weise wie die Biegefeder (90) erstreckt und eine Mehrzahl von Hohlräumen (101) aufweist, die jeweils derart geformt sind, dass sie mit mindestens einem Zahn (91) der Biegefeder (90) zusammenwirken, um die Biegefeder (90) und somit die Platte (9) in einer gewünschten Position innerhalb des Trägerrahmens (1) zu arretieren.

2. Spritze (200) nach Anspruch 1,
**dadurch gekennzeichnet, dass** die Biegefeder (90) dazu ausgebildet ist, mindestens eine erste Verriegelungsposition einzunehmen, in der die Nadel (3) teilweise aus dem Ende (121) des Kopfes (12) herausragt, ohne dass in der anfänglichen Positionierungsphase der Röhrchenampulle (2) eine Fluidverbindung besteht,
eine zweite Verriegelungsposition einzunehmen, die dazu geeignet ist, die Injektion eines Teils des in der Röhrchenampulle (2) enthaltenen Fluids unter teilweiser Entleerung der Röhrchenampulle (2) zu ermöglichen, und
eine dritte Verriegelungsposition einzunehmen, die geeignet ist für den vollständigen Wiedereintritt der Nadel (3) ins Innere des Rahmens (1) und anschließend erneute Austritte der Nadel (3) aus dem Ende (121) des Kopfes (12) zur Durchführung weiterer Injektionen, um die Röhrchenampulle (2) vollständig zu entleeren.

3. Spritze (200) nach Anspruch 2,
**dadurch gekennzeichnet, dass** der Kopf (12) einen zylindrischen Hohlraum aufweist, in dem mindestens eine vordere Feder (60, 61) zumindest teilweise untergebracht ist, und dass der Boden (13) einen zylindrischen Hohlraum aufweist, der dazu ausgebildet ist, mindestens eine hintere Feder (62, 63) zumindest teilweise aufzunehmen.

4. Spritze (200) nach Anspruch 3,
**dadurch gekennzeichnet, dass** der zylindrische Hohlraum des Kopfes (12) dazu ausgebildet ist, zwei vordere Federn (60, 61), eine bewegliche Lochplatte (123) und eine Endhülse (124) mindestens teilweise aufzunehmen, wobei die bewegliche Lochplatte (123) zwischen den beiden vorderen Federn (60, 61) angeordnet ist, wobei das vordere Ende einer vorderen Feder (61) in der Endhülse (124) untergebracht ist, die an einem inneren Vorderteil des Kopfes (12) unter dem Ende (121) festgelegt ist, und wobei die bewegliche Lochplatte (123) dazu ausgebildet ist, eine derartige Position einzunehmen, dass die Endhülse (124) an einer Hülse anliegt (125), die an der beweglichen Lochplatte (123) auf der Seite der Endhülse (124) angeordnet ist.

5. Spritze (200) nach Anspruch 3 oder 4,
**dadurch gekennzeichnet, dass** der zylindrische Hohlraum des Bodens (13) dazu ausgebildet ist, zwei hintere Federn (62, 63) und eine bewegliche Lochplatte (131), die zwischen den beiden hinteren Federn (62, 63) angeordnet ist, zumindest teilweise aufzunehmen, wobei das hintere Ende der ersten hinteren Feder (63) an der beweglichen Lochplatte (131) festgelegt ist, während das vordere Ende der ersten hinteren Feder (63) dazu ausgebildet ist, das hintere Ende der Röhrchenampulle (2) stützend aufzunehmen, wobei die zweite hintere Feder (62) zwischen der beweglichen Lochplatte (131) und einem inneren hinteren Stützteil des Bodens (13) um die Durchgangsöffnung des Bodens (13) herum angeordnet ist.

6. Spritze (200) nach einem der vorhergehenden Ansprüche,
**dadurch gekennzeichnet, dass** die Nadel (3) ein konisches Ende (31), einen zylindrischen Bereich (32) mit einem Durchmesser, der größer als der eigentliche Durchmesser der Nadel (3) ist, und einen kegelstumpfförmigen Bereich (33) aufweist, wobei das konische Ende (31) mit einer Ebene quer zu der Längsachse der Nadel (3) geschnitten ist, um ein Durchstechen der Membran (7) mit geringem Druck zu ermöglichen.

7. Spritze (200) nach einem der vorhergehenden Ansprüche,
**dadurch gekennzeichnet, dass** die Biegefeder (90) an einem hinteren Ende einen Knopf (92) aufweist, der es dem Benutzer ermöglicht, die Biegefeder (90) zu biegen.

8. Spritze (200) nach Anspruch 7,
**dadurch gekennzeichnet, dass** mindestens ein Zahn (91) der Biegefeder (90) in Entsprechung zu dem Knopf (92) positioniert ist.

## Revendications

1. Seringue (200) avec aiguille (3) de type rétractable à actionnement assisté, appropriée pour l'administration multiple intraveineuse ou intramusculaire de fluides et pour le retour de l'aiguille (3) afin d'éviter les piqûres accidentelles, comprenant un châssis de support (1) de forme creuse, adapté pour supporter un flacon tubulaire (2) contenant le fluide à administrer, et une tige (4) se terminant par une poignée (41), supportée, de manière coulissante, par le châssis de support (1) et adaptée pour déplacer, de manière axiale, un piston plongeur (8) du flacon tubulaire (2) logé dans un corps transparent (21) du flacon tubulaire (2) qui a également un diaphragme (7) réalisé avec un matériau caoutchouteux pouvant être percé par l'aiguille (3),
dans laquelle le châssis de support (1) a un corps (11) adapté pour supporter le flacon tubulaire (2) dans une cavité (50) du corps (11) et qui a une tête (12) et un fond (13) aux extrémités,
dans laquelle la tête (12) comprend une extrémité (121) adaptée pour supporter l'aiguille (3) qui est adaptée pour sortir d'un trou de l'extrémité (121) de la tête (12) et le fond (13) comprend un trou débouchant pour que la tige (4) coulisse,
dans laquelle au moins la tête (12) ou le fond (13) ont une cavité cylindrique dans laquelle au moins un ressort (60-63) est logé au moins partiellement,
dans laquelle la seringue (200) comprenant en outre :
une plaque (9) logée, de manière coulissante, dans la direction axiale dans le corps (11), adjacente au diaphragme (7) du flacon tubulaire (2), qui est orthogonale par rapport à la direction axiale de mouvement de la tige (4) et percée au centre pour le passage de l'aiguille (3),
**caractérisée en ce que** la seringue (200) comprend en outre :
au moins un ressort de flexion (90) qui s'étend axialement et qui est fixé à la plaque (9) au niveau d'une extrémité avant (93), dans laquelle le ressort de flexion (90) est flexible dans la direction radiale et dans la direction circonférentielle pour une fonction de blocage/déblocage du mouvement axial du ressort de flexion (90),
un support (100) pour le blocage axial du ressort de flexion (90), dans lequel le support (100) est solidaire avec le corps (11), s'étend de manière axiale d'une manière similaire vers le ressort de flexion (90) et a une pluralité de cavités (101), chacune formée pour se mettre en prise avec au moins une dent (91) du ressort de flexion (90) afin de bloquer le ressort de flexion (90) et par conséquent la plaque (9) dans une position souhaitée à l'intérieur du châssis de support (1).

2. Seringue (200) selon la revendication 1, **caractérisée en ce que** le ressort de flexion (90) est adapté pour adopter au moins :
une première position de blocage dans laquelle l'aiguille (3) fait partiellement saillie de l'extrémité (121) de la tête (12), sans raccordement de fluide dans la phase de positionnement initiale du flacon tubulaire (2),
une deuxième position de blocage appropriée pour permettre l'injection d'une partie du fluide contenu dans le flacon tubulaire (2) avec le vidage partiel du flacon tubulaire (2), et
une troisième position de blocage appropriée pour le retour complet de l'aiguille (3) à l'intérieur du châssis (1) et successivement de nouvelles sorties de l'aiguille (3) de l'extrémité (121) de la tête (12) pour l'exécution d'injections ultérieures pour vider complètement le flacon tubulaire (2).

3. Seringue (200) selon la revendication 2, **caractérisée en ce que** la tête (12) comprend une cavité cylindrique dans laquelle au moins un ressort avant (60, 61) est logé au moins partiellement, et **en ce que** le fond (13) comprend une cavité cylindrique adaptée pour loger au moins partiellement au moins un ressort arrière (62, 63).

4. Seringue (200) selon la revendication 3, **caractérisée en ce que** la cavité cylindrique de la tête (12) est adaptée pour loger au moins partiellement deux ressorts avant (60, 61), une plaque perforée mobile (123) et un manchon d'extrémité (124), dans laquelle la plaque perforée mobile (123) est intercalée entre les deux ressorts avant (60, 61), dans laquelle l'extrémité avant d'un ressort avant (61) est logée dans le manchon d'extrémité (124) qui est fixé sur une partie interne avant de la tête (12) sous l'extrémité (121), et dans laquelle la plaque perforée mobile (123) est adaptée pour aller dans une position, moyennant quoi le manchon d'extrémité (124) vient en butée sur un manchon (125) placé sur la plaque perforée mobile (123) sur le côté du manchon d'extrémité (124).

5. Seringue (200) selon la revendication 3 ou 4, **caractérisée en ce que** la cavité cylindrique du fond (13) est adaptée pour loger au moins partiellement deux ressorts arrière (62, 63) et une plaque perforée mobile (131) placée entre les deux ressorts arrière (62, 63), dans laquelle l'extrémité arrière du premier ressort arrière (63) est fixée sur la plaque perforée mobile (131), alors que l'extrémité avant du premier ressort arrière (63) est adaptée pour recevoir, en appui, l'extrémité arrière du flacon tubulaire (2), dans laquelle le second ressort arrière (62) est intercalé entre la plaque perforée mobile (131) et une partie de support arrière interne du fond (13) autour du trou débouchant du fond (13).

6. Seringue (200) selon l'une quelconque des revendications précédentes, **caractérisée en ce que** l'aiguille (3) comprend une extrémité conique (31), une partie cylindrique (32) avec un diamètre supérieur au diamètre dominant de l'aiguille (3), et une partie tronconique (33), dans laquelle l'extrémité conique (31) est coupée avec un plan transversal à l'axe longitudinal de l'aiguille (3) pour permettre de percer le diaphragme (7) avec une basse pression.

7. Seringue (200) selon l'une quelconque des revendications précédentes, **caractérisée en ce qu'**au niveau d'une extrémité arrière, le ressort de flexion (90) a un bouton (92) qui permet à l'utilisateur de fléchir le ressort de flexion (90).

8. Seringue (200) selon la revendication 7, **caractérisée en ce qu'**au moins une dent (91) du ressort de flexion (90) est positionnée en correspondance du bouton (92).
